(19) Europäisches Patentamt
European Patent Office
Office européen des brevets

(11) Veröffentlichungsnummer: **0 151 473 B1**

(12) # EUROPÄISCHE PATENTSCHRIFT

(45) Veröffentlichungstag der Patentschrift: **27.01.88**

(51) Int. Cl.[4]: **C 07 C 69/612**, C 07 C 67/30

(21) Anmeldenummer: **85101052.0**

(22) Anmeldetag: **01.02.85**

(54) **Verfahren zur Herstellung von Estern optisch aktiver 2-Arylalkansäuren.**

(30) Priorität: **08.02.84 DE 8404336**

(43) Veröffentlichungstag der Anmeldung:
**14.08.85 Patentblatt 85/33**

(45) Bekanntmachung des Hinweises auf die Patenterteilung:
**27.01.88 Patentblatt 88/4**

(84) Benannte Vertragsstaaten:
**BE CH DE FR GB IT LI NL**

(56) Entgegenhaltungen:
**BULLETIN OF THE CHEMICAL SOCIETY OF JAPAN, Band 52, Nr. 8, August 1979, Seiten 2377-2382, T. NAKAJIMA et al. "Alkylation of Benzene with Optically Active 3-Chloro-l-butanol, 3-Chloro-butanoic Acid and their Esters"**
**PATENT ABSTRACTS OF JAPAN, Band 7, Nr. 77 (C-159)[1222], 30 März 1983; & JP - A - 58 8045 (SEITETSU KAKAKU KOGYO) 18.01.1983**
**CHEMICAL ABSTRACTS, Band 83, Nr. 9, 1. September 1975, Seite 638, Spalte 1, Abstract Nr. 78972b, Columbus, Ohio, US; T. NAKAJIMA et al. "Asymmetric induction in the Friedel-Crafts reaction with (+)-1,2-epoxybutane and (-)-2-chloro-1-butanol", & BULL. CHEM. SOC. JPN. 1975, 48(3), 960-965**

(73) Patentinhaber: **BASF Aktiengesellschaft, Carl-Bosch-Strasse 38, D-6700 Ludwigshafen (DE)**

(72) Erfinder: **Fritz, Gerhard, Dr., Limburgstrasse 23, D-6701 Dannstadt-Schauernheim (DE)**
Erfinder: **Eggersdorfer, Manfred, Dr., Hannongstrasse 18, D-6710 Frankenthal (DE)**
Erfinder: **Siegel, Hardo, Dr., Hans-Purrmann-Allee 25, D-6720 Speyer (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein neues Verfahren zur Herstellung von Estern optisch aktiver 2-Aryl-alkansäuren der allgemeinen Formel I

$$\genfrac{}{}{0pt}{}{Ar}{R^1}{>}CH\text{-}COOR^2 \qquad \text{I}$$

in der Ar einen iso- oder heterocyclischen Arylrest bedeutet und in der $R^1$ und $R^2$ für $C_1$-$C_4$-Alkylgruppen stehen.

Optisch aktive Verbindungen dieses Typs wurden, wie allgemein bekannt ist, bisher in der Weise hergestellt, dass man das I-Racemat in das Racemat der entsprechenden Säure überführt, dieses mit einem optisch aktiven Amin zu einem diastereomeren Salzpaar umsetzt und letzteres durch Kristallisation in seine beiden Komponenten zerlegt, aus denen man die optisch aktiven Säuren gewinnt, die dann anschliessend wieder verestert werden.

Da diese klassische Methode der Racematspaltung jedoch evident umständlich ist, lag der Erfindung die Bereitstellung eines einfacheren Verfahrens zur Herstellung der optisch aktiven Verbindungen I als Aufgabe zugrunde.

Demgemäss wurde ein Verfahren zur Herstellung der eingangs definierten optisch aktiven 2-Arylalkansäuren I gefunden, welches dadurch gekennzeichnet ist, dass man einen optisch aktiven Ester der allgemeinen Formel II

$$\genfrac{}{}{0pt}{}{X}{R^1}{>}CH\text{-}COOR^2 \qquad \text{II}$$

in der X Chlor, Brom oder einen Rest Y-$SO_2$-O- bedeutet, in welchem Y für Chlor oder einen Kohlenwasserstoffrest steht, nach Friedel-Crafts mit einer aromatischen Verbindung ArH (III) umsetzt.

Dieses Verfahren ist äusserst bemerkenswert, da es bekannt war, dass die Umsetzung optisch aktiver Verbindungen nach Friedel-Crafts von einer vollständigen Racemisierung des eingesetzten Enantiomeren begleitet wird, worauf beispielsweise Nakajima et al in ihrer Arbeit «Alkylation of Benzene with Optically Active 3-Chloro-1-butanol...» (Bull. Chem. Soc. Jap. 52(8), 1979, S. 2377 f.) einleitend hinweisen. Hier wird zwar über eine Ausnahme von dieser Regel berichtet, die jedoch damit erklärt wird, dass die Reaktion über einen Sechsring verläuft, der sich intermediär aus dem Chlorbutanol und $AlCl_3$ bildet. Im vorliegenden Falle ist die Bildung eines derartigen Sechsringes jedoch nicht möglich.

Die optisch aktiven Ausgangsverbindungen II sind, soweit sie nicht bekannt sind, auf einfache Weise und unter Erhalt der optischen Aktivität aus den entsprechenden Estern der optisch aktiven Hydroxysäuren IIa

$$\genfrac{}{}{0pt}{}{HO}{R^1}{>}CH\text{-}COOR^2 \qquad \text{IIa}$$

erhältlich, indem man diese z.B. mit Thionylchlorid, Thionylbromid oder auch in Gegenwart einer Base mit einem Sulfonsäurechlorid Y-$SO_2$-Cl umsetzt. Als Reste Y werden hierbei Chlor, $C_1$-$C_4$-Alkyl, insbesondere Methyl, Benzyl, Phenyl und 4-Methylphenyl bevorzugt.

Besondere Bedeutung haben diejenigen Ausgangsverbindungen II, welche sich von der Milchsäure ($R_1$ = Methyl) ableiten, zumal deren Enantiomeren, z.B. durch mikrobiologische Verfahren, leicht zugänglich sind. Unter den übrigen Verbindungen II ist noch diejenige hervorzuheben, in der $R^1$ die Isopropylgruppe bedeutet. Die erfindungsgemässe Umsetzung als solche hängt von der Art des Restes $R^1$ indes praktisch nicht ab, so dass sich die Art des Restes nach den Wirkstoffeigenschaften der Produkte I oder von deren Folgeprodukten richtet, die man letztlich herzustellen wünscht.

Die Art des Restes $R^2$ hat keinen erkennbaren Einfluss auf das erfindungsgemässe Verfahren, so dass man aus praktischen Gründen in der Regel von den einfachsten Estern II, nämlich dem Methylester und dem Ethylester ausgeht.

Als Beispiele für iso- und heterocyclische Verbindungen III seien die Stammkörper Benzol, Naphthalin, Anthracen, Peneanthren, Pyridin, die Pyrazine, Chinolin und Thiophen genannt. Diese und weitere Verbindungen III können Substituenten tragen, z.B. Halogen und Alkyl-, Alkoxy-, Aryl-, Carbalkoxy-, Acyloxy-, Cyan- und Nitrogruppen. Weiterhin können z.B. nicht-aromatische iso- oder heterocyclische Ringe an die aromatischen Stammkörper annelliert sein.

Da die Umsetzung von II mit III im allgemeinen glatt und auch vollständig gelingt, empfiehlt es sich, diese Ausgangsverbindungen in äquimolaren Mengen einzusetzen. Ist III eine billige Substanz wie Benzol und Toluol, kann man diese auch in molarem Überschuss über II sowie auch als Lösungsmittel verwenden.

Als sonstige Lösungsmittel eignen sich Flüssigkeiten, die sich unter den Reaktionsbedingungen inert verhalten, also z.B. Schwefelkohlenstoff, Nitroalkane, Nitrobenzol, Acetonitril, Diethylether, Dimethylformamid sowie auch halogenierte Kohlenwasserstoffe wie 1,2-Dichlorethan, 1,1,2,2-Tetrachlorethan, Chloroform und Tetrachlorkohlenstoff, sofern unter relativ milden Reaktionsbedingungen gearbeitet wird.

Als Friedel-Crafts-Katalysatoren kommen die üblichen für diese Reaktion verwendeten Lewis-Säuren in Betracht, also in erster Linie $AlCl_3$, $ZnCl_2$, $FeCl_3$ und $BF_3$. Da diese Verbindungen stöchiometrisch in die Umsetzung eingehen, setzt man sie zweckmässigerweise in äquimolaren oder leicht überschüssigen Mengen zu II ein.

Die Reaktionstemperaturen liegen im allgemeinen zwischen 0° und 100°C, vorzugsweise zwischen 0° und 25°C. Im allgemeinen wird man bei Normaldruck oder leicht erhöhtem Druck arbeiten.

Im übrigen nimmt man die Umsetzung wie üblich vor, etwa wie es in der JP-OS 8045/1983 für die Umsetzung von racemischen α-Chlorsulfonyloxypropionsäureestern mit Benzol zu racemischen α-Phenylpropionsäureestern beschrieben ist.

Auch die Aufarbeitung der Reaktionsgemische bietet keine Besonderheiten, so dass nähere Ausführungen hierzu entbehrlich sind.

Die optisch aktiven Verfahrensprodukte I, die in hoher Ausbeute und hoher optischer Reinheit anfallen, sind teils selbst wichtige physiologische Wirkstoffe und teils Vorstufen hierzu und bereichern allgemein die Synthesemöglichkeiten auf diesem Gebiet.

So dienen beispielsweise S-(p-Hydroxyphenyl)-α--isopropylessigsäureester als wichtige Ausgangsmaterialien für synthetische Pyrethroide und d-(+)--6-Methoxy-α-methyl-2-naphthalinessigsäure als Antiphlogisticum und Antirheumaticum.

*Beispiel*

Herstellung von S-(+)-α-Phenylpropionsäureethylester

Eine Lösung aus 94 g (1,2 mol) Benzol und 80 g (0,6 mol) $AlCl_3$ wurde im Laufe einer Stunde bei 0° bis 10°C mit 43,3 g (0,2 mol) S-(+)-α-Chlorosulfonyloxypropionsäureethylester versetzt, noch 2 Stunden bei 20°C gerührt und danach wie üblich auf den S-(+)-α-Phenylpropionsäureethylester aufgearbeitet. Die Ausbeute betrug 76% und die optische Ausbeute 95%.

Der α-Chlorosulfonyloxypropionsäureethylester wurde durch Umsetzung von S-(−)-Milchsäureethylester mit Sulfurylchlorid bei 0°C in praktisch quantitativer Ausbeute hergestellt.

**Patentanspruch**

Verfahren zur Herstellung von Estern optisch aktiver 2-Aryl-alkansäuren der allgemeinen Formel I

$$\genfrac{}{}{0pt}{}{Ar}{R^1}{>}CH\text{-}COOR^2 \qquad I$$

in der Ar einen iso- oder heterocyclischen Arylrest bedeutet und in der $R^1$ und $R^2$ für $C_1$-$C_4$-Alkylgruppen stehen, dadurch gekennzeichnet, dass man einen optisch aktiven Ester der allgemeinen Formel II

$$\genfrac{}{}{0pt}{}{X}{R^1}{>}CH\text{-}COOR^2 \qquad II$$

in der X Chlor, Brom oder einen Rest $Y$-$SO_2$-O- bedeutet, in welchem Y für Chlor oder einen Kohlenwasserstoffrest steht, nach Friedel-Crafts mit einer aromatischen Verbindung ArH (III) umsetzt.

**Claim**

A process for the preparation of an ester of an optically active 2-arylalkanoic acid of the formula I

$$\genfrac{}{}{0pt}{}{Ar}{R^1}{>}CH\text{-}COOR^2 \qquad I$$

where Ar is an isocyclic or heterocyclic aryl radical and $R^1$ and $R^2$ are each $C_1$-$C_4$-alkyl, wherein an optically active ester of the formula II

$$\genfrac{}{}{0pt}{}{X}{R^1}{>}CH\text{-}COOR^2 \qquad II$$

where X is chlorine, bromine or a radical $Y$-$SO_2$-O-, in which Y is chlorine or a hydrocarbon radical, is subjected to a Friedel-Crafts reaction with an aromatic compound ArH (III).

**Revendication**

Procédé de préparation d'esters d'acides aryl-2-alcanoïques optiquement actifs de la formule générale I

$$\genfrac{}{}{0pt}{}{Ar}{R^1}{>}CH\text{-}COOR^2 \qquad I$$

dans laquelle Ar représente un groupe aryle iso- ou hétéro-cyclique et $R^1$ et $R^2$ désignent des radicaux alkyle en $C_1$ à $C_4$, caractérisé en ce que l'on fait réagir un ester optiquement actif de la formule générale II

$$\genfrac{}{}{0pt}{}{X}{R^1}{>}CH\text{-}COOR^2 \qquad II$$

dans laquelle X désigne un atome de chlore ou de brome ou un groupe $Y$-$SO_2$-O-, dans lequel Y désigne un atome de chlore ou un groupe hydrocarboné, selon Friedel-Crafts avec un composé aromatique ArH (III).